# EUROPEAN PATENT APPLICATION

(11) **EP 4 792 792 A1**
(43) Date of publication of application: **19.08.2026**
(21) Application number: 25158467.8
(22) Date of filing: 18.02.2025
(51) Int. Cl.: A61F 13/15, A61F 13/49, A61F 13/494

(54) **ABSORBENT ARTICLES WITH BARRIER LEG CUFFS**

(71) Applicant: The Procter & Gamble Company, Cincinnati, OH 45202 (US)
(72) Inventor: COELHO, Filipa Alexandra Macedo Tavares, 65824 Schwalbach am Taunus (DE); ROSATI, Rodrigo, 65824 Schwalbach am Taunus (DE); MARONEZE, Mateus, 65824 Schwalbach am Taunus (DE); LESZCZYNSKI, Szymon, 65824 Schwalbach am Taunus (DE); JENNEWEIN, Marc, 65824 Schwalbach am Taunus (DE)
(74) Representative: P&G Patent Germany

(57) **Abstract**

Absorbent articles having a liquid permeable topsheet, a liquid impermeable backsheet, an absorbent core disposed at least partially between the topsheet and the backsheet, and a pair of barrier leg cuffs is provided. Each barrier leg cuff is formed from a cuff material comprising hydrophobic cellulosic fibers.

## Description

### FIELD

The present disclosure is generally directed to absorbent articles (e.g., diapers, adult incontinence articles, feminine hygiene pads, disposable absorbent inserts, and the like) having barrier leg cuffs formed from hydrophobic cellulosic fibers.

### BACKGROUND

Absorbent articles, such as diapers, adult incontinence articles, feminine hygiene pads, disposable absorbent inserts, and the like are often used for containing urine and/or other bodily exudates. To effectively contain bodily exudates, the articles should provide a snug fit around the waist and legs of a wearer. Current absorbent article designs frequently include a barrier leg cuff to assist in the reduction of leakage of bodily exudates. Barrier leg cuffs typically include an inner cuff that is disposed inboard of the absorbent member of the absorbent article and is meant to contain bodily exudates within the absorbent article and proximate the absorbent member so it can be absorbed. The inner cuff may have an elastic strand disposed therein such that the inner cuff may form a gasket along the leg of the wearer to help inhibit bodily exudates from leaking out of the absorbent article.

Absorbent articles typically comprise a wide variety of different materials, including plastic films, foam, superabsorbent polymer particles, natural materials such as cellulose fiber, etc. Due to the presence of bodily exudates and the cost and difficulty associated with separating the component materials and rendering them suitable for reuse, used absorbent articles have typically been excluded from the stream of waste products for differential collection and recycling. As a result, most used absorbent articles are currently disposed of as regular, undifferentiated waste. However, there have been processes developed to separate the different materials and to recycle or biodegrade the different components of absorbent articles with particular focus on super-absorbent polymers.

For other functional parts of the absorbent article such as the barrier leg cuffs, there is still a need to reduce the environmental impact, for example by employing materials that are biodegradable. Hence, the environmental impact of barrier leg cuffs and of absorbent articles with barrier leg cuffs should be improved, while maintaining the desired functional properties such as leakage protection.

### SUMMARY

An absorbent article comprising a liquid permeable topsheet; a liquid impermeable backsheet; an absorbent core disposed at least partially between the topsheet and the backsheet; and a pair of barrier leg cuffs is provided. Each barrier leg cuff is formed from a cuff material. The cuff material comprises hydrophobic cellulosic fibers. Each barrier leg cuff may be formed from a single web of cuff material comprising hydrophobic cellulosic fibers. The hydrophobic cellulosic fibers should be biodegradable such that the environmental impact of the absorbent article is reduced. It was found that by choosing hydrophobic cellulosic fibers as cuff material, a high level of biodegradability, and thus compostability, and the required water resistance of the cuff material can be achieved.

Further, an absorbent article comprising a liquid permeable topsheet; a liquid impermeable backsheet; an absorbent core disposed at least partially between the topsheet and the backsheet; and a pair of barrier leg cuffs is provided. Each barrier leg cuff is formed from a cuff material. The cuff material has a water resistance of at least 6 mbar, preferably at least 8 mbar, more preferably at least 9 mbar, even more preferably at least 10 mbar or even at least 13 mbar according to the Water Resistance Test method disclosed herein. The cuff material has a biodegradability of at least 80 %, preferably at least 90 %, more preferably at least 95 %, even more preferably at least 98 % or even at least 99 % according to the Biodegradability Test method disclosed herein.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin; and

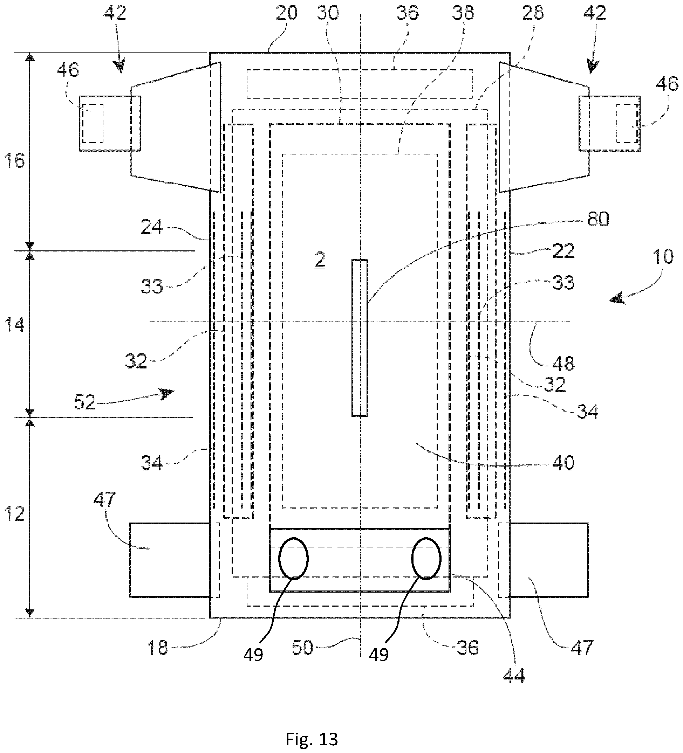

Fig. 13 is a plan view of an example absorbent article in the form of a taped diaper, garment-facing surface facing the viewer, in a flat laid-out state.

### DETAILED DESCRIPTION

Various non-limiting forms of the present disclosure will now be described to provide an overall understanding of the principles of the structure, function, manufacture, and use of the absorbent articles with barrier leg cuffs having a backfolded inner cuff disclosed herein. One or more examples of these non-limiting forms are illustrated in the accompanying drawings. Those of ordinary skill in the art will understand that the absorbent articles with barrier leg cuffs having a backfolded inner cuff described herein and illustrated in the accompanying drawings are non-limiting example forms. The features illustrated or described in connection with one non-limiting form may be combined with the features of other non-limiting forms. Such modifications and variations are intended to be included within the scope of the present disclosure.

### General Description of an Absorbent Article

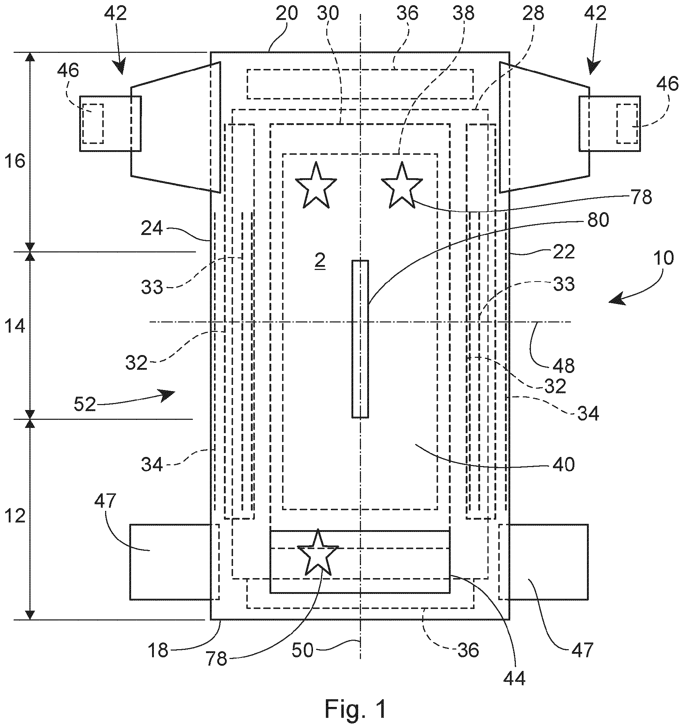

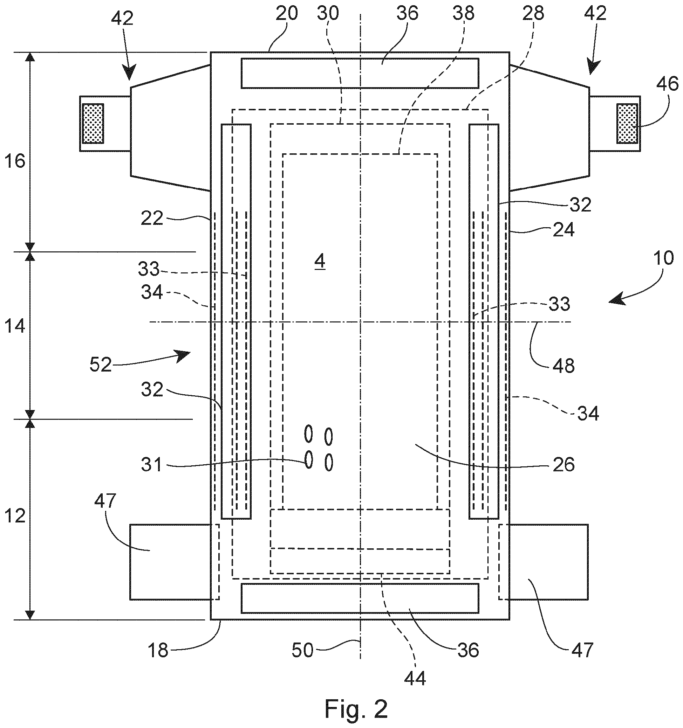

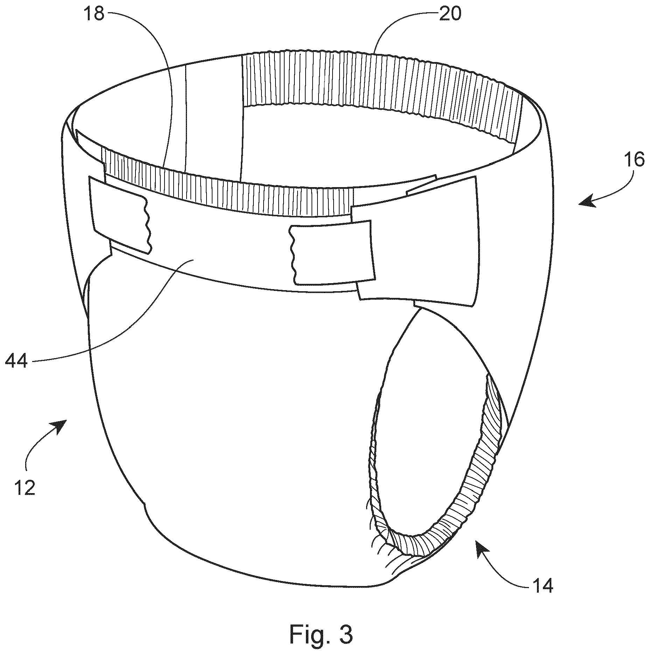

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of absorbent articles, including diapers, adult incontinence articles, pant-style articles, sanitary napkins, and absorbent inserts, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be approximately 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially between the topsheet 26 and the backsheet 28. The absorbent article 10 may also comprise one or more pairs of barrier leg cuffs 32 as described further herein, one or more elastic waistbands 36, and/or one or more acquisition materials 38. The acquisition material or materials 38 may be positioned between the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

The absorbent article may comprise a secondary fastening system comprising a secondary fastening component 210 and a secondary receiving component 212 that are operatively engageable to further secure the article about the wearer. The secondary fastening component 210 may be disposed in the front waist region 12, and the secondary f receiving component 212 may be disposed in the back waist region 16. Addition of a secondary fastening system can provide a greater surface area for fastening, and thereby de-concentrate lateral tensile forces communicated through the fastening location(s) as the rear waist region is pulled toward the front waist region, and vice versa, when the diaper is worn. In addition, having two distinct fastening locations reduces the tendency of the front portion of the article to pivot (i.e., pivot around the single fastening location of the primary fastening system). Further, the secondary system helps to create a line of tension closer to the front waist edge, which may reduce the likelihood of folding or flipping over of the front waist edge during wear. Further still, the secondary system may create an anchoring geodesic to direct forces from the crotch region to over the hips in order to prevent sagging during wearer. The secondary system may also help to secure the front ear or combination belt structures in place during wear. Each of the foregoing can serve to provide for more effective and durable fastening and less longitudinal and/or lateral flexing, sagging and/or wrinkling of the diaper materials about the fastening areas during wear.

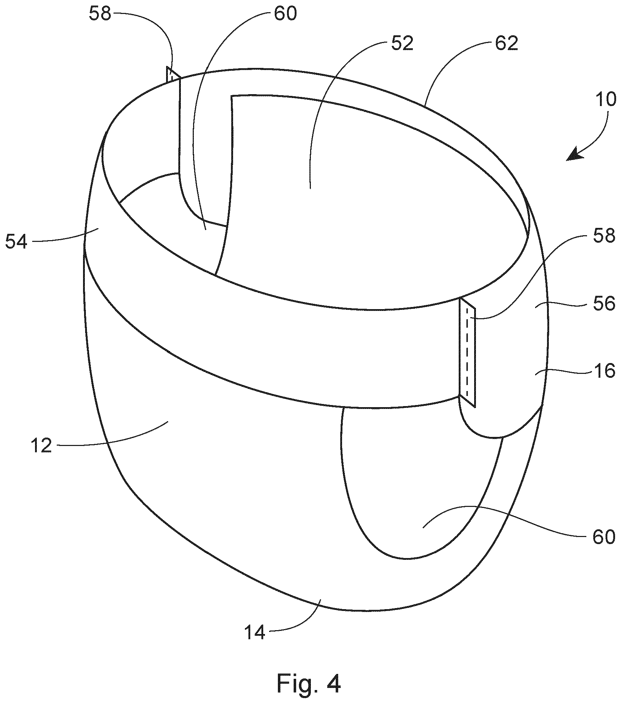

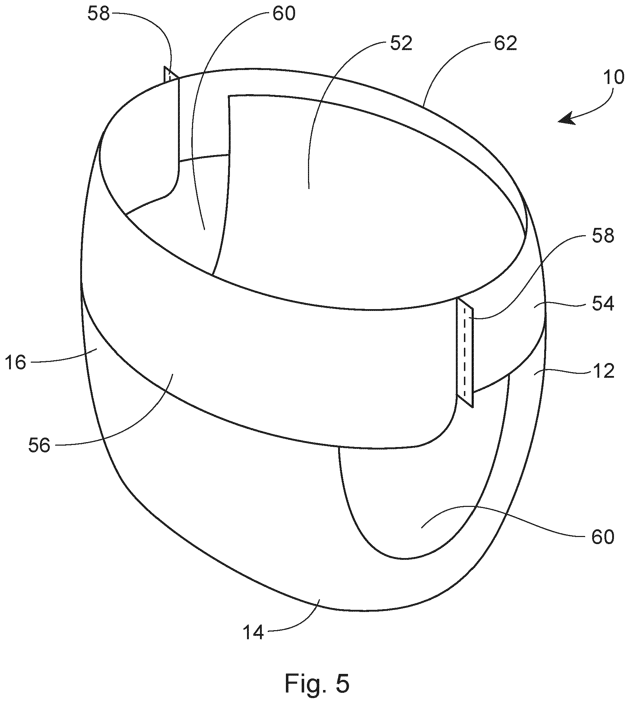

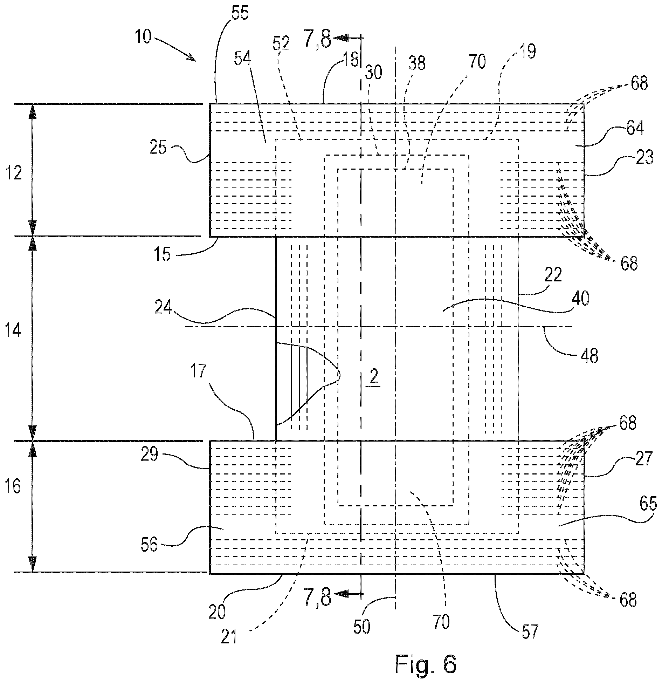

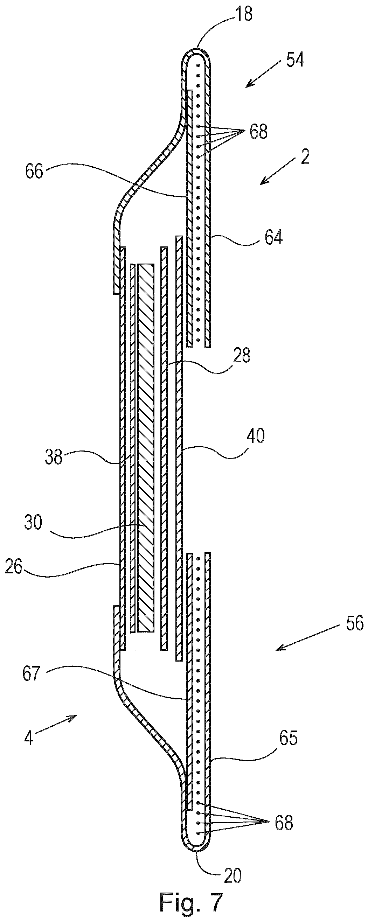

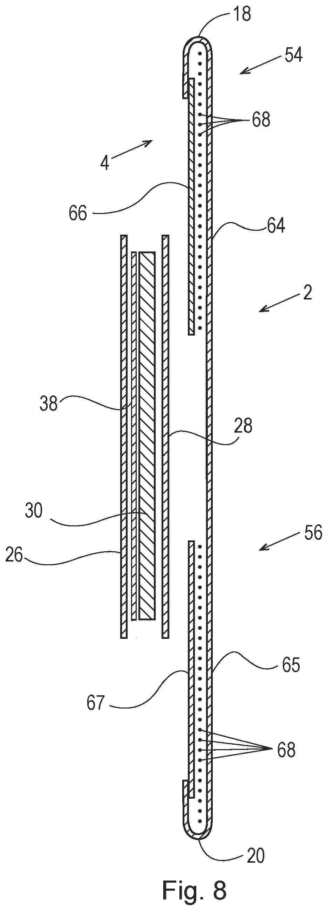

In other instances, the absorbent article may be in the form of a pant (pant-style) having permanent or refastenable side seams. Suitable refastenable seams are disclosed in U.S. Pat. Appl. Pub. No. 2014/0005020 and U.S. Pat. No. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

In another form, the absorbent article may be an insert 2500 for use with a reusable outer cover 2502, as shown in Fig. 25. The insert 2500 may be disposable or reusable. The reusable outer cover 2502 may comprise a woven or other material and may be configured as a pant or a taped diaper. In the taped context, the reusable outer cover 2502 may comprise a fastening system used to join a front waist region of the reusable outer cover to a back waist region. The fastening system may comprise snaps, buttons, and/or hooks and loops, for example. The insert 2500 may comprise a liquid permeable topsheet, a liquid impermeable backsheet, and an absorbent core positioned at least partially between the topsheet and the backsheet. One or more acquisition and/or distribution materials may be positioned between the topsheet and the absorbent core. The insert 2500 may comprise one or more pairs of leg cuffs and may be free of ears, side panels, and/or waistbands. In some instances, a nonwoven material may be positioned on a garment-facing side of the backsheet. A garment-facing surface of the insert 2500 may be attached to a wearer-facing surface of the reusable outer cover 2502 via adhesives, hook and loop fasteners, or other methods of joinder. An example insert and reusable outer cover system is disclosed in U.S. Patent No. 9,011,402, issued on April 21, 2015, to Roe, et al. The insert or the reusable outer cover may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or may alternatively run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet. The topsheet may comprise a one, or more, layer hydroentangled material with or without apertures. The topsheet may comprise a variable basis weight nonwoven material.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material.

### Absorbent Core

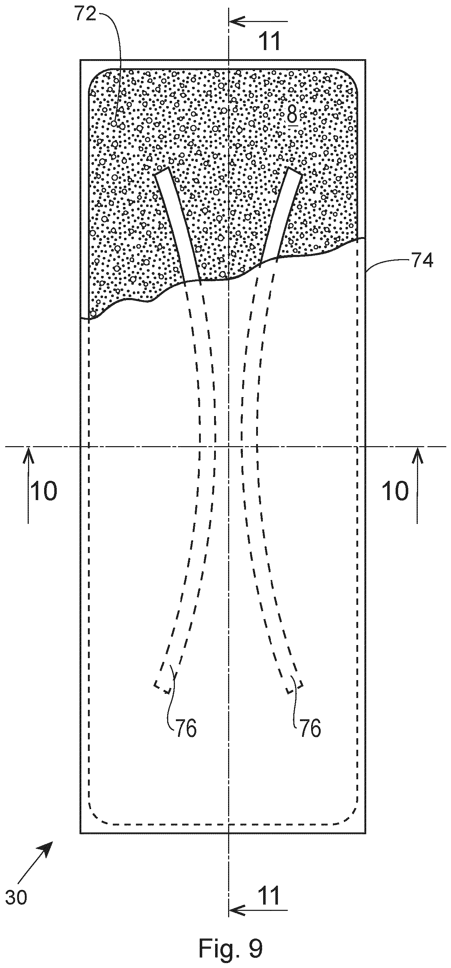

As used herein, the term "absorbent core" 30 refers to a component of the absorbent article 10 disposed in the article for absorbing and containing liquid such as urine received by the absorbent article. The absorbent core thus typically has a high absorbent capacity. An example absorbent core 30 is schematically shown in Figs. 9-11. The absorbent core comprises an absorbent material 72, that is typically enclosed within or sandwiched between a core bag 74.

The core wrap may be a single material that is folded and attached to itself, or it may comprise a separate top layer and bottom layer that may be bonded or otherwise joined together. The absorbent material typically comprises superabsorbent particles which are optionally mixed with cellulose fibers. As used herein, "absorbent core" does not include any acquisition-distribution systems, topsheet, or backsheet of the absorbent article.

The example absorbent core 30 shown in isolation in Figs. 9-11 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 9. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "hourglass" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 g/g to 40 g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in U.S. Pat. No. 5,599,335 (Goldman), EP1,447,066 (Busam), WO95/11652 (Tanzer), U.S. Pat. Appl. Pub. No. 2008/0312622A1 (Hundorf), WO2012/052172 (Van Malderen). In particular, the SAP printing technology as disclosed in U.S. Pat. Appl. Pub. No. 2006/024433 (Blessing), U.S. Pat. Appl. Pub. No. 2008/0312617 and U.S. Pat. Appl. Pub. No. 2010/0051166A1 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate such as a carded nonwoven layer, having a porous structure into which SAP particles have been deposited, may also be used in present disclosure.

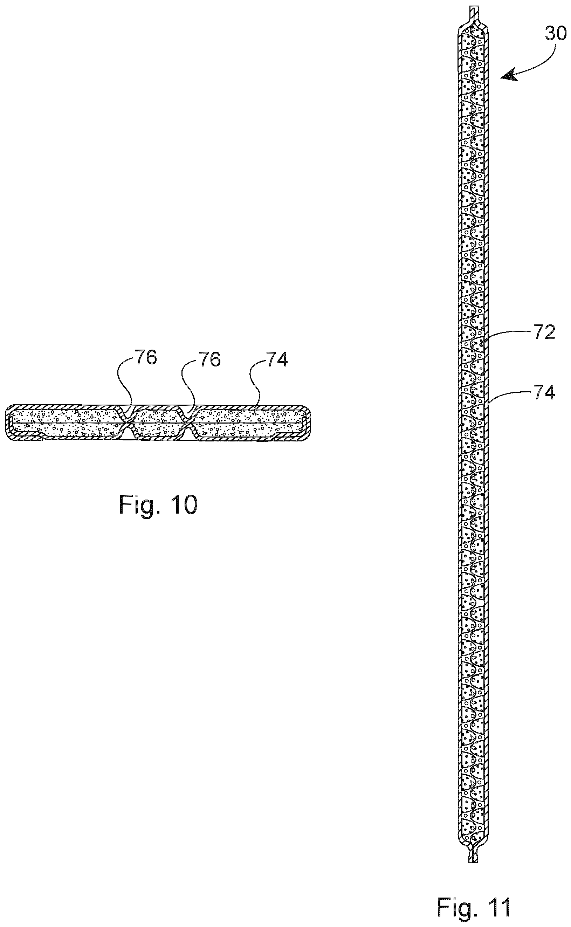

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area, as illustrated in Figs. 10-11. As for example taught in U.S. Pat. Appl. Pub. No. 2008/312,622 A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channels 76, which are areas of the absorbent layer substantially free of absorbent material within the absorbent material layer. The top side of the core wrap may be advantageously bonded to the bottom side of the core by adhesive, mechanical or ultrasonic bonding through these material-free areas. Example disclosures of such channels in an airfelt-free core can be found in WO2012/170778 (Rosati et al.) and US2012/0312491 (Jackels). Channels may of course also be formed in absorbent cores comprising a mix of cellulose fibers and SAP particles. These channels may embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

### Elastic Waist Feature

Referring to Figs. 1 and 2, the absorbent article 10 may comprise one or more elastic waist features 36. The one or more elastic waist features 36 may comprise a waistband or a waist cuff. The elastic waist feature 36 may be positioned on the garment-facing surface 2 or the wearer-facing surface 4. Alternatively, the elastic waist feature 36 may be positioned between the topsheet 26 and the backsheet 28. As an example, a first elastic waist feature 36 may be present in the front waist region 12 near the front waist edge 18 and a second elastic waist feature 36 may be present in the back waist region 16 near the back waist edge 20. The elastic waist feature 36 may aid in sealing the absorbent article 10 around a waist of a wearer and at least inhibiting bodily exudates from escaping the absorbent article 10 through the waist opening circumference. In some instances, an elastic waist feature may fully surround the waist opening circumference of an absorbent article 10. The elastic waist feature 36 may comprise an elastic film joined to the topsheet 26 and a nonwoven material covering the elastic film. In other instances, the elastic waist feature 36 may comprise an elastic film sandwiched between two nonwoven materials. The elastic film may be ultrasonically bonded, or otherwise bonded or attached, to the one or more nonwoven materials. The one or more nonwoven materials may be hydroentangled. The elastic film and/or the nonwoven materials may be preactivated (i.e., activated prior to being joined together) or the formed elastic film/nonwoven laminate may be activated post laminate formation.

### Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, formed films, apertured formed films, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2.

### Masking Layer

One or more masking layers or materials may be provided in the absorbent articles 10. A masking layer may be a layer that provides a cushiony feel when the absorbent article is touched from the garment-facing surface 2 or the wearer-facing surface 4. The masking layer may "mask" a grainy feel potentially caused by the absorbent material 72, such as superabsorbent polymers. The masking layer may "mask" bodily exudates from being visible when viewing the wearer-facing surface 4 or the garment-facing surface 2 of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the wearer-facing side of the core bag 74. The masking layer may be a separate material positioned intermediate the garment-facing side of the core bag 74 and the liquid impermeable backsheet 28.

### Stretch Laminate

Various elements of the absorbent articles 10 described herein may comprise a stretch laminate. For example, any of the belts 54 and 56 and/or any of the ears 42, 47 may comprise a stretch laminate as described hereinafter. The waistband(s) 36 may also comprise a stretch laminate. Such laminates may include an elastomeric layer that provides extensibility to the laminate and one or more outer layers that is less stretchable but suitable for providing durability and desirable tactile properties. In this way, the laminate permits a component of an absorbent article to closely and comfortably contact the wearer, while providing desirable exterior qualities.
Figs. 23A-F are cross-sectional views of various stretch laminates 90. As shown in Fig. 23A, a stretch laminate 90 may comprise a first cover layer 100 and an elastomeric film layer 300 joined via one or more ultrasonic bonds 400. The elastomeric film layer 300 may have one or more skins, such as a first skin 301 providing first surface, and a second skin 302 providing second surface. As shown in Fig. 23B, a stretch laminate 90 may comprise a first cover layer 100 and a second cover layer 200 with an elastomeric film layer 300 sandwiched therebetween in a facing relationship to both the first cover layer 100 and the second cover layer 200. All three layers may be joined via one or more ultrasonic bonds 400. As shown in Figs. 23C and 23D, all, or a portion of, the first cover layer 100 may comprise one or more layers, such as a first layer 101 and second layer 102, which may have the same composition or different compositions. Similarly, all or a portion of, the second cover layer 200 may comprise one or more layers, such as a first layer 201 and second layer 202, which may have the same composition or different compositions. As shown in Figs. 23E and 23F, a portion of the first cover layer 100 or the second cover layer 200 may be folded over to provide a multi-layered structure on all or a portion of the opposite side of stretch laminate 90.

Elastomeric film layer 300 of stretch laminate 90 may include a single layer or multiple layers of one or more materials that are elastically extensible. The elastically extensible material(s) may be between about 10 µm and about 100 µm, or between about 20 µm and about 60 µm, or between about 30 µm and about 50 µm, or in some embodiments, about 40 µm, in thickness. The elastically extensible material(s) may comprise an elastomeric polyolefin, and in some embodiments, a polyolefin (POE) blown film.

The elastically extensible material may comprise modifying resins. The elastically extensible material may comprise a variety of additives. Suitable additives including, but are not limited to, stabilizers, antioxidants, and bacteriostats may be employed to prevent thermal, oxidative, and biochemical degradation of the elastically extensible material. Additives may account for about 0.01% to about 60% of the total weight of the elastically extensible material. In other embodiments, the composition comprises from about 0.01% to about 25%. In other suitable embodiments, the elastically extensible material comprises from about 0.01% to about 10% by weight, of additives.

The ultrasonic bonds 400 preferably eliminate the need for any adhesives, but adhesives may be employed to join the layers 100, 200, 300 of the stretch laminate 90. Adhesives may be selected from any adhesives known to provide suitable attachment between elastomeric film layer 300 and cover layers 100, 200. In some embodiments, the adhesive may be a hot melt adhesive with a basis weight of less than about 15 gsm.

Elastomeric film layer 300 may be mechanically pre-activated before attachment to at least one cover layer 100, 200. For example, elastomeric film layer 300 may be pre-activated by being stretched transversely to its web direction by more than 50% (i.e., strain > 50%). In some embodiments, an expansion by about 100% to about 500% occurs in relation to the starting width of elastomeric film layer 300. In alternate embodiments, elastomeric film layer 300 may be stretched in the web direction, stretched a direction other than the web direction or transverse to the web direction, or a combination of directions. The term "stretching" is to point to the fact that the expansion of elastomeric film layer 300 is not completely reversible and that a non-elastic fraction results in the film having a larger width following pre-activation (i.e., the elastomeric film does not have 100% recovery, and therefore has a percent set value). After expansion, elastomeric film layer 300 retracts and has a width that may be larger by about 10% to about 30% in relation to a starting width of the film. In other words, after the pre-activation expansion and retraction detailed below, elastomeric film layer 300 may exhibit a set of about 10% to about 30%.

According to various embodiments in which elastomeric film layer 300 includes both an elastically extensible material and at least one skin disposed on the elastically extensible material, the pre-activation process may physically alter these materials differently, for example, because these materials have different elasticity and recovery properties. During pre-activation, the skin 301 and/or 302 and the elastically extensible material are similarly stretched (i.e., put under similar strain). However, after stretching, the skin and the elastically extensible material will retract and recover differently (i.e., have different set values). In comparison with the elastically extensible material, the skin is less elastic and therefore will have less recovery after stretching, a.k.a., a higher set value. The skin is also much thinner than the elastically extensible material, so when the thicker elastically extensible material retracts and recovers after pre-activation stretching, it will force the attached skin to retract with it. But because the skin cannot recover as much as the elastically extensible material, the skin buckles and wrinkles. Accordingly, the cross-sectional profile and the top view appearance of elastomeric film layer 300 are modified after a pre-activation process.

In some configurations, it may be desirable to provide a stretch laminate or an element of an absorbent article 10 (such as a belt or an ear) comprising a stretch laminate with different zones of performance characteristics. Such enhanced properties may include breathability, softness, strength, thickness, uniformity in rugosities, modulus, aesthetic enhancements, tear resistance, combinations of any of the foregoing and/or zones having differing values of any of the foregoing features. According to various embodiments, the stretch laminates and/or elements comprising stretch laminates may comprise: embossing, apertures, perforations, slits, melted material or coatings, compressed material, secondary bonds that are disposed apart from the chassis attachment bond, plastic deformation, and folds.

Figure 24 is an exploded perspective view of an exemplary ear 42, 47 comprising a stretch laminate 90 schematically illustrating exemplary surface modifications. The surface modification(s) may be made to a precursor material prior to lamination. One or more surface modifications 800 (also referred to as morphological features) are illustrated in one or more layers of the laminate. Surface modifications 800 may comprise embossing 802, cuts (e.g., apertures, perforations, slits), melted material or coatings, compressed material 808, plastic deformation 810 (e.g., activation stripes 812), folds 814, post-formation bonds 816 (e.g., adhesive bonds, pressure bonds, thermal bonds, and/or ultrasonic bonds applied after the substrate is formed) and combinations thereof. Surface modifications may be formed after the initial formation of the substrate itself, thereby forming a modified substrate. In other words, thermal bonding fibers to produce a nonwoven is not considered a surface modification. Creating bonds on the nonwoven once it has been formed is considered a surface modification. Additionally, or alternatively, surface modifications 800 on different layers may work together to provide an enhanced property.

One or more structural features may be formed on the laminate after initial bonding of the layers. Structural features may comprise embossing, cuts (e.g., apertures, perforations, slits), melted material or coatings, compressed material, plastic deformation (e.g., activation stripes), folds, secondary bonds (e.g., adhesive bonds, pressure bonds, thermal bonds, and/or ultrasonic bonds applied after the initial bonding of the laminate) and combinations thereof. A structural feature or a surface modification may be in the form a design element.

It is to be appreciated that combinations of one or more surface modifications and one or more structural features may be employed. It is also to be appreciated that certain cover layer substrates may be selected for their amenability to surface modifications and/or structural features. By way of nonlimiting example, a carded nonwoven may be selected if mechanically activating the substrate and/or laminate. Lower modulus materials, such as polyethylene-based materials, may be more suitable for modification through laser energy.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate number of absorbent articles per package. By packaging the absorbent articles under compression, caregivers can easily handle and store the packages, while also providing distribution savings to manufacturers owing to the size of the packages.

### Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g., oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Sanitary Napkin

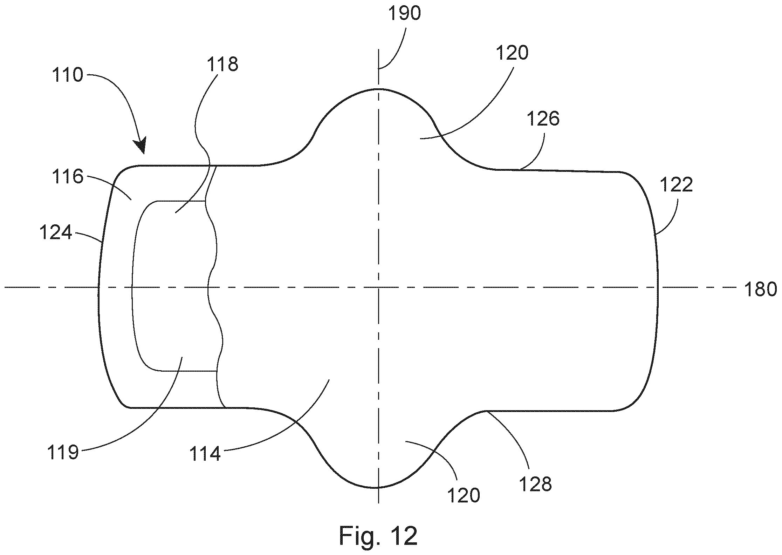

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

### Barrier Leg Cuffs

The absorbent article 10 of the present disclosure comprises a pair of barrier leg cuffs 32. The barrier leg cuffs 32 may extend at least partially, or completely, between the front end edge 18 and the back end edge 20 of the absorbent article 10 on opposite sides of the central longitudinal axis 50 and may be at least present in the crotch region 14. The absorbent article 10 of the present disclosure may comprise one or more pairs of leg elastics 34. The barrier leg cuffs 32 may be positioned laterally inboard of leg elastics 34. Each barrier leg cuff 32 may be formed by a piece of cuff material which is bonded to the absorbent article 10 so it can extend upwards from a wearer-facing surface 4 of the absorbent article 10 and provide improved containment of body exudates approximately at the junction of the torso and legs of the wearer. In particular, each barrier leg cuff 32 of the pair of barrier leg cuffs may be formed from a single web of cuff material.

The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

The single web of cuff material forming the barrier leg cuff 32 may be folded laterally outward to form a backfold between a proximal portion and an inner distal portion of the barrier leg cuff 32. The creation of the backfold results in the formation of a backfolded inner cuff. The cuff web of cuff material may be further folded laterally inward to form a backfold folded edge and laterally outward to form an inner folded edge. The backfold folded edge may be disposed laterally outboard of the inner folded edge when the absorbent article is in a flat, uncontracted configuration. The backfolded inner cuff may comprise an inner elastic element disposed proximate the inner folded edge and a backfold elastic element disposed proximate the backfold folded edge. The inner elastic element and the backfold elastic element may each comprise a single elastic member or may each comprise a plurality of elastic members. The inner elastic element and the backfold elastic element may extend the entire length of each backfolded inner cuff. For example, where the backfolded inner cuff extends from the front end edge 18 to the back end edge 20 of the absorbent article 10, the inner elastic element and backfold elastic element may likewise extend the length of the absorbent article between the front end edge 18 and the back end edge 20. The inner elastic element and the backfold elastic element may not extend the entire length of each backfolded inner cuff. The inner elastic element and the backfold elastic element may extend only a partial length of the backfolded inner cuff, for example only in the crotch region 14, or only in the crotch region 14 and the back waist region 16. The backfolded inner cuff comprising two different elastic elements (the inner elastic element and the backfolded elastic element) spaced apart from each other, may form two separate gasketing points between the inner cuff and the body of the wearer, resulting in reduced leaking of bodily exudates out of the absorbent article during wear. The inner elastic element and the backfold elastic element may be attached to the backfolded inner cuff along the entire length of the backfolded inner cuff, or the elastic elements may be attached to only a portion of the backfolded inner cuff. The inner elastic element and the backfold elastic element may be attached to the backfolded inner cuff by any suitable means, such as with adhesive, thermal bonding, pressure bonding, ultrasonic bonding, and combinations thereof.

### Bio-Based Content for Components

Components of the absorbent articles described herein may at least partially be comprised of bio-based content as described in U.S. Pat. Appl. No. 2007/0219521 A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Recyclability of Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

### Biodegradability of Absorbent Articles

The absorbent article may have a Biodegradability of at least 80 %, preferably at least 90 %, more preferably at least 95 %, even more preferably at least 98 % or even at least 99 % according to the Biodegradability Test method disclosed herein.

To achieve a high biodegradability, as many components of the absorbent article should be biodegradable.

A suitable topsheet may be manufactured from a wide range of biodegradable materials, such as woven or nonwoven materials of natural fibers (e.g. softwood pulp, hardwood pulp, cotton, bamboo pulp, abaca pulp, hydrophilic viscose, hydrophilic Lyocell, hemp, flax, kapok, kenaf, jute, cupro, ramie, sisal, and combinations thereof.) and/or biodegradable polymeric fibers (e.g. PBAT, PLA, PHA, PHB, PBS, PBSA, PCL, cellulose acetate, starch, PVOH, etc).

The absorbent article may comprise biodegradable acquisition materials 38. The biodegradable acquisition materials 38 may comprise one or more nonwoven materials, cellulosic materials, cross-linked cellulosic materials, air laid cellulosic nonwoven materials, spunlace materials, or combinations thereof. In particular, the biodegradable acquisition materials 38 may comprise cellulosic materials, which is cross-linked with citric acid. The cellulose-based acquisition material may be combined with an acquisition layer: the acquisition layer may be manufactured from a wide range of biodegradable materials, such as woven or nonwoven materials of natural fibers (e.g. softwood pulp, hardwood pulp, cotton, bamboo pulp, abaca pulp, hydrophilic viscose, hydrophilic Lyocell, hemp, flax, kapok, kenaf, jute, cupro, ramie, sisal, etc.) and/or biodegradable polymeric fibers (e.g. PBAT, PLA, PHA, PHB, PBS, PBSA, PCL, cellulose acetate, starch, PVOH, etc).

The absorbent article may comprise an absorbent core 30 comprising biodegradable superabsorbent particles ("SAP"). Biodegradable superabsorbent particles may comprise polysaccharide polymers and derivatives of polysaccharide polymers, such as polymers based on cellulose or starch; polyaspartates and the respective salts thereof, polyglutamate and the respective salts thereof, gamma- polyglutamate and the respective salts thereof,.

Preferred biodegradable SAP may be polysaccharide polymers and derivatives of polysaccharide polymers, such as polymers based on cellulose or starch. A polysaccharide is a carbohydrate that can be decomposed by hydrolysis into two or more molecules of monosaccharides. Polymers suitable for use as SAP include cellulose-based polymers such as carboxymethylcellulose. An example is Spongel^{®}, manufactured by Magic S.r.L, Oleggio, Italy. Spongel^{®} is mainly a cellulose-based material, cross-linked and/or reinforced by naturally derived inorganic fillers. Suitable starch-based superabsorbent polymers and methods for their synthesis are described in DE 19619680, published on November 20th 1997. Other suitable polysaccharides include polyalginates and carrageenan. Carrageenan is a generic description of a group of sulphated polysaccharides. Different polysaccharide SAP may be used in combination. Other preferred biodegradable SAP may be copolymers of starch and acrylic acid, charge modified starch-based biopolymer, starch grafted polyacrylic acid copolymer sodium salt, polypropiolactone based SAP, starch grafted with carboxylic groups, polyacrylic acid crosslinked with starch-based crosslinkers, styrene maleic acid copolymers, wheat gluten based SAP, itaconic acid and salts thereof.

Other diaper components, such as masking layers, core wraps, outer cover material, front and back ears, belt materials, landing zones may be manufactured from a wide range of biodegradable materials, such as woven or nonwoven materials of natural fibers (e.g. softwood pulp, hardwood pulp, cotton, bamboo pulp, abaca pulp, hydrophilic viscose, hydrophilic Lyocell, hemp, flax, kapok, kenaf, jute, cupro, ramie, sisal, etc .) and/or biodegradable polymeric fibers (e.g. PBAT, PLA, PHA, PHB, PBS, PBSA, PCL, cellulose acetate, starch, PVOH, etc).Further, biodegradable adhesives, such as starch-based adhesives, biodegradable hooks or tapes and biodegradable elastics could be employed.

### Cuff material

The cuff material comprises hydrophobic cellulosic fibers. The hydrophobic cellulosic fibers may comprise or consist of cotton, hemp, kapok, sisal, viscose, lyocell or combinations thereof. Preferably, the hydrophobic cellulosic fibers may comprise or consist of viscose, lyocell or combinations thereof. The cellulosic fibers may be hydrophobic without treatment such kapok, sisal or unbleached cotton.

The term "hydrophilic" describes fibers or surfaces of fibers, which are wettable by aqueous fluids (e.g., aqueous body fluids) deposited on these fibers. Hydrophilicity and wettability are typically defined in terms of contact angle and the surface tension of fluids as they pass through a material. A fiber or surface of a fiber is said to be wetted by an aqueous fluid (i.e., hydrophilic) when either the contact angle between the fluid and the fiber, or its surface, is less than 90 degrees, or when the fluid tends to spread spontaneously across the surface of the fiber. Conversely, a fiber or surface of the fiber is considered to be "hydrophobic" if the contact angle is greater than 90 degrees and the fluid does not spread spontaneously across the surface of the fiber.

The hydrophobic cellulosic fibers may be treated to be hydrophobic. In particular, the hydrophobic cellulosic fibers may be treated with one or more biodegradable hydrophobic additive. Examples for biodegradable hydrophobic additives are alkyl ketene dimers (AKDs), cotton seed oil, and combinations thereof. The hydrophobic cellulosic fibers may be surface-treated to be hydrophobic and/or the hydrophobic additive may be incorporated into the fibers.

The cuff material may have a basis weight of from 25 gsm to 60 gsm, preferably from 30 gsm to 50 gsm according to the Basis Weight Test method disclosed herein. Each barrier leg cuff 32 of the pair of barrier leg cuffs may be formed from a single web of cuff material and the single web may have a basis weight of from 25 gsm to 60 gsm, preferably from 30 gsm to 50 gsm according to the Basis Weight Test method disclosed herein. By choosing such a basis weight, a good balance between biodegradability (generally requiring lower basis weights) and water resistance (generally requiring higher basis weights) can be achieved.

The cuff material may have a water resistance of at least 6 mbar, preferably at least 8 mbar, more preferably at least 9 mbar, even more preferably at least 10 mbar or even at least 13 mbar according to the Water Resistance Test method disclosed herein. A water resistance of 6 mbar can be achieved with petroleum based materials and was found to be the minimum requirement for a cuff material to provide a sufficient leakage control when employed as cuff in an absorbent article.

The cuff material may have a Biodegradability of at least 80 %, preferably at least 90 %, more preferably at least 95 %, even more preferably at least 98 % or even at least 99 % according to the Biodegradability Test method disclosed herein. By such a value, the environmental impact of the cuff material is reduced.

The hydrophobic cellulosic fibers may be treated with one or more bio-based and/or biodegradable hydrophobic additive such that cuff material has a water resistance of at least 6 mbar, preferably at least 8 mbar, more preferably at least 9 mbar, even more preferably at least 10 mbar or even at least 13 mbar according to the Water Resistance and has a Biodegradability of at least 80 %, preferably at least 90 %, more preferably at least 95 %, even more preferably at least 98 % or even at least 99 % according to the Biodegradability Test method disclosed herein.

The cuff material may exhibit one or more of the following mechanical properties.

The cuff material may have a machine direction (MD) tensile peak strength of at least 6.0 N/cm, preferably at least 7.0 N/cm, more preferably at least 8.0 N/cm, even more preferably at least 9.0 N/cm or even at least 9.0 N/cm according to the Tensile Strength method disclosed herein.

The cuff material may have a cross-machine direction (CD) tensile peak strength of at least 2.0 N/cm, preferably at least 2.5 N/cm, more preferably at least 3.0 N/cm, even more preferably at least 3.5 N/cm or even at least 4.0 N/cm according to the Tensile Strength method disclosed herein.

The cuff material may have a machine direction (MD) peak elongation of at least 15 %, preferably at least 20 %, more preferably at least 25 % or even at least 30 % according to the Tensile Strength method disclosed herein.

The cuff material may have a cross-machine direction (CD) peak elongation of at least 20 %, preferably at least 40 %, more preferably at least 60 %, even more preferably at least 60 % or even at least 100 % according to the Tensile Strength method disclosed herein.

Each barrier leg cuff may be bonded directly to the topsheet 26 and/ or the backsheet 28. In particular, each barrier leg cuff may be adhesively bonded to the topsheet 26 and/ or the backsheet 28. Cellulosic materials may create a stronger bond than non-cellulosic, polymeric materials due to the fiber structure.

### Test methods

### Biodegradability Test

Biodegradability is determined according to DIN EN ISO 14855-1 - 2012. The Biodegradability is determined as percentage of conversion of carbon in the sample to carbon dioxide in the aerobic biodegradation of the sample on exposure to a controlled-composting environment under laboratory conditions, at thermophilic temperatures.

The total carbon content is determined as the sum of the carbon-content of all materials, which are present in the sample at a concentration of equal to or more than 1 weight-% based on the total weight of the dry mass of the sample. The carbon content of the materials can be either determined via elemental analysis or by calculation if the chemical composition of the material is well established.

The cumulative amount of produced carbon dioxide is determined over a period of 6 months. After the 6 months, the percentage of biodegradability is determined via dividing the biodegraded carbon content, as determined from the produced carbon dioxide, by the total carbon content determined for the initial sample.

### Water Resistance Test

The Water Resistance value is a measure of the liquid barrier property (or liquid impermeability) of a material. Specifically, the test described herein measures the hydrostatic pressure the material will support when water penetration occurs. The TexTest Hydrostatic Head Tester FX3000 (available from Advanced Testing Instruments, Corp., Spartanburg, SC) is used.

The test method conforms to NWSP 080.6.R0 (15). For this test, gradually increasing pressure is applied to a defined sample portion until water penetrates through the sample.

The test is conducted in a laboratory environment typically about 23°C ± 2 °C and a relative humidity of about 30 ± 15%.

The sample is clamped over the top of the column fixture, using an appropriate gasketing material (o-ring style) to prevent side leakage during testing. In order to determine the Water Resistance of the cuff material, a sample of one layer of cuff material is oriented such that a surface of the cuff material faces the water column during the test. The area of water contact with the sample is equal to the cross sectional area of the water column, which equals 100 cm². In either instance, water is pumped into the water column at a rate to achieve a pressure increase of 10 mbar/min or 60 mbar/min . Thus, the sample is subjected to a steadily increasing water pressure on one surface. When water penetration appears on three locations on the other surface of the sample, the pressure at which the third penetration occurs is recorded. If water immediately penetrates the sample (i.e., the sample provided no resistance), a zero reading is recorded. For each material, three specimens are tested and the results are averaged and reported in mbars.

### Basis Weight Test

Basis weight of the materials comprising may be determined by several available techniques, but a simple representative technique involves taking an absorbent article or other consumer product, removing any elastic which may be present and stretching the absorbent article or other consumer product to its full length. A punch die having an area of 45.6 cm² is then used to cut a piece of the cuff material from the absorbent article or other consumer product in a location which avoids to the greatest extent possible any adhesive which may be used to fasten the material to any other layers which may be present and potentially removing the cuff material from other layers (using cryogenic spray, such as Cyto-Freeze, Control Company, Houston, Texas, if needed). The sample is then weighed, and the weight is divided by the area of the punch die yielding the basis weight of the cuff material. Results are reported as a mean of 5 samples to the nearest 0.1 g/m² (gsm).

### Tensile Strength Test

The cross-machine direction (CD) tensile peak strength and cross-machine direction (CD) peak elongation of a specimen is measured according to WSP 110.4-09 with conditions below.
Preload: 0.1N
Test Speed: 100 mm/min
Sample Width: 50 mm
Sample length: sufficiently longer than gauge length
10 Gauge Length: 100 mm

The machine direction (MD) tensile peak strength and machine direction (MD) peak elongation of a specimen is measured according to WSP 110.4-09 with conditions below.
Preload: 0.1N
Test Speed: 100 mm/min
Sample Width: 50 mm
15 Sample length: sufficiently longer than gauge length
Gauge Length: 100 mm

### Examples

Comparative Example 1: Spunbond nonwoven made of polypropylene with a basis weight of 13 gsm. Comparative Example 1 represents a standard cuff material.

Comparative Example 2: Spunbond nonwoven comprising 100% polylactic acid fibers, a biodegradable thermoplastic polyester, fibers treated with a biodegradable hydrophobic additive with a basis weight of 20 gsm. Comparative Example 2 represents a standard biodegradable nonwoven material

Example 1: Spunlace nonwoven comprising 100% viscose fibers treated with a bio-based hydrophobic additive with a basis weight of 40 gsm. The biodegradable hydrophobic additive is incorporated into the fiber matrix during the spinning process.

Example 2: Spunlace nonwoven made of 100% lyocell fibers surface-treated with alkyl ketene dimer (AKD) additive (less than 0.1 weight-% based on the total weight of the fibers) with a basis weight of 33 gsm.

Comparative Examples 1 and 2 and Examples 1 and 2 were tested for their Water Resistance according to the Water Resistance Test and Biodegradability according to the Biodegradability Test methods disclosed herein.

**Table 1**

| **Material** | **Water Resistance [mbar]** |
|---|---|
| Comp. Ex. 1 | 9 |
| Comp. Ex. 2 | 1 |
| Ex. 1 | 14 |
| Ex. 2 | 6 |

The tests showed that Examples 1 and 2 meet the Water Resistance requirements to be employed as cuff material, while being biodegradable. In particular, Example 1 exhibits the best Water Resistance of the tested materials.

Comparative Example 1 meets the Water Resistance requirements but is not biodegradable, while Comparative Example 2 is biodegradable but does not meet the Water Resistance requirement.

The biodegradable materials Comparative Example 2 and Examples 1 and 2 were tested for their mechanical properties according to the Tensile Strength Test method disclosed herein.

**Table 2**

| **Material** | **Tensile strength MD peak [N/cm]** | **Tensile strength CD peak [N/cm]** | **Elongation MD peak [%]** | **Elongation CD peak [%]** |
|---|---|---|---|---|
| Comp. Ex. 2 | 5.4 | 1.8 | 10 | 15 |
| Ex. 1 | 10.6 | 4.5 | 31 | 107 |
| Ex. 2 | | | 28 | 118 |

The biodegradable material Example 2 was included as cuff material in diapers having other biodegradable components according to Table 3 to yield biodegradable taped diapers Examples D1, D2 and D3. All glues comprised by the diapers were regular hotmelts non-biodegradable. All elastic materials comprised by the diapers were regular non-biodegradable elastic materials. The elastic materials and glues made up a total of 5.5 - 6.3 weight-% based on the total weight of the diaper.

**Table 3**

| | **Options** | | |
|---|---|---|---|
| **Component** | **D1** | **D2** | **D3** |
| Backsheet Cover | 40 gsm Viscose (100% by weight) nonwoven (6.6g/diaper) | 40 gsm Viscose (100% by weight) nonwoven (6.6g/diaper) | 40 gsm Viscose (100% by weight) nonwoven (6.6g/diaper) |
| Breathable backsheet film | 30 gsm corn starch based PLA (5.0g/diaper) | 30 gsm corn starch based PLA (5.0 g/diaper) | 30 gsm corn starch based PLA (5.0 g/diaper) |
| Tape | 45 gsm bamboo based (0.4 g/diaper) | 45 gsm bamboo based (0.4 g/diaper) | 45 gsm bamboo based (0.4 g/diaper) |
| Back ear | 65gsm bamboo based (0.4 g/diaper) | 65gsm bamboo based (0.4 g/diaper) | 65gsm bamboo based (0.4 g/diaper) |
| Dusting Layer Tissue | 18 gsm Wood fiber (0.8 g/diaper) | 18 gsm Wood fiber (0.8 g/diaper) | 18 gsm Wood fiber (0.8 g/diaper) |
| SAP | Carboxy-methyl-cellulose - based (6 g/diaper) | Carboxy-methyl-cellulose - based (10 g/diaper) | Carboxy-methyl-cellulose - based (15 g/diaper) |
| Fluff pulp (mixed with SAP) | Chlorine free bleached fluff pulp (20 g/diaper) | Chlorine free bleached fluff pulp (20 g/diaper) | Chlorine free bleached fluff pulp (20 g/diaper) |
| Core Cover Tissue | 18 gsm Wood fiber (1.3 g/diaper) | 18 gsm Wood fiber (1.3 g/diaper) | 18 gsm Wood fiber (1.3 g/diaper) |
| Acquisition Layer | 35 gsm PLA based (0.6 g/diaper) | 35 gsm PLA based (0.6 g/diaper) | 35 gsm PLA based (0.6 g/diaper) |
| Topsheet | 40gsm Viscose (100% by weight) nonwoven (2.8 g/diaper) | 40gsm Viscose (100% by weight) nonwoven (2.8 g/diaper) | 40gsm Viscose (100% by weight) nonwoven (2.8 g/diaper) |
| Cuffs | 40gsm Example 2 nonwoven (7.8 g/diaper) | 40gsm Example 2 nonwoven (7.8 g/diaper) | 40gsm Example 2 nonwoven (7.8 g/diaper) |
| Waist Elastic nonwoven* | 0. 8g/diaper | 0. 8g/diaper | 0. 8g/diaper |
| Elastics* | 0.3g/diaper | 0.3g/diaper | 0.3g/diaper |
| Glues* | 2.4g/diaper | 2.4g/diaper | 2.4g/diaper |

| | | | |
|---|---|---|---|
| *made with conventional non-biodegradable materials | | | |

The Biodegradability of Examples D1-D3 is determined according to the Biodegradability Test methods disclosed herein.

### Contemplated Examples

1. An absorbent article (10) comprising:
   a liquid permeable topsheet (26);
   a liquid impermeable backsheet (28);
   an absorbent core (30) disposed at least partially between the topsheet (26) and the backsheet (28); and
   a pair of barrier leg cuffs (32), each barrier leg cuff (32) formed from a cuff material, wherein the cuff material has a water resistance of at least 6 mbar, preferably at least 8 mbar, more preferably at least 9 mbar, even more preferably at least 10 mbar or even at least 13 mbar according to the Water Resistance Test method disclosed herein and
   wherein the cuff material has a Biodegradability of at least 80 %, preferably at least 90 %, more preferably at least 95 %, even more preferably at least 98 % or even at least 99 % according to
   the Biodegradability Test method disclosed herein.
2. The absorbent article (10) of example 1, wherein the cuff material comprises hydrophobic cellulosic fibers.
3. The absorbent article (10) of example 2, wherein the hydrophobic cellulosic fibers comprise or consist of viscose, lyocell or combinations thereof.
4. The absorbent article (10) of examples 2 or 3, wherein the hydrophobic cellulosic fibers are treated to be hydrophobic.
5. The absorbent article (10) of example 4, wherein the hydrophobic cellulosic fibers are treated with biodegradable hydrophobic additive.
6. The absorbent article (10) of any one of examples 1-5, wherein the cuff material has a basis weight of from 25 gsm to 60 gsm, preferably from 30 gsm to 50 gsm according to the Basis Weight Test method disclosed herein.
7. The absorbent article (10) of any one any one of examples 1-6, wherein the cuff material has a machine direction (MD) tensile peak strength of at least 6.0 N/cm, preferably at least 7.0 N/cm, more preferably at least 8.0 N/cm, even more preferably at least 9.0 N/cm or even at least 9.0 N/cm according to the Tensile Strength Test method disclosed herein.
8. The absorbent article (10) of any one of any one of examples 1-7, wherein the cuff material has a cross-machine direction (CD) tensile peak strength of at least 2.0 N/cm, preferably at least 2.5 N/cm, more preferably at least 3.0 N/cm, even more preferably at least 3.5 N/cm or even at least 4.0 N/cm according to the Tensile Strength Test method disclosed herein.
9. The absorbent article (10) of any one of any one of examples 1-8, wherein the cuff material has a machine direction (MD) peak elongation of at least 15%, preferably at least 20 %, more preferably at least 25 % or even at least 30 % according to the Tensile Strength Test method disclosed herein.
10. The absorbent article (10) of any one of examples 1-9, wherein the cuff material has a cross-machine direction (CD) peak elongation of at least 20 %, preferably at least 40 %, more preferably at least 60 %, even more preferably at least 60 % or even at least 100 % according to the Tensile Strength Test method disclosed herein.
11. The absorbent article (10) of any one of examples 1-10, wherein the absorbent article has a Biodegradability of at least 70 %, preferably at least 80 %, more preferably at least 90 %, even more preferably at least 95 %, even more preferably at least 98 % or even at least 99 % according to the Biodegradability Test method disclosed herein.
12. The absorbent article (10) of any one of examples 1-11, wherein the topsheet (26) has a Biodegradability of at least 80 %, preferably at least 90 %, more preferably at least 95 %, even more preferably at least 98 % or even at least 99 % according to the Biodegradability Test method disclosed herein.
13. The absorbent article (10) of any one of examples 1-12, wherein the backsheet (28) has a Biodegradability of at least 80 %, preferably at least 90 %, more preferably at least 95 %, even more preferably at least 98 % or even at least 99 % according to the Biodegradability Test method disclosed herein.
14. The absorbent article (10) of any one of examples 1-13, wherein the absorbent core (30) has a Biodegradability of at least 80 %, preferably at least 90 %, more preferably at least 95 %, even more preferably at least 98 % or even at least 99 % according to the Biodegradability Test method disclosed herein.
15. The absorbent article (10) of any one of examples 1-14, wherein the absorbent article comprises a biodegradable acquisition material (38), preferably wherein the biodegradable acquisition materials (38) is a crosslinked cellulosic material, in particular a cellulosic material crosslinked with citric acid.
16. The absorbent article (10) of any one of examples 1-15, wherein the absorbent article comprises biodegradable superabsorbent particles.
17. The absorbent article (10) of example 16, wherein the biodegradable superabsorbent particles comprise polysaccharide polymer, derivatives of polysaccharide polymers, polyaspartate; polyglutamate; and/ or the respective salts thereof.
18. The absorbent article (10) of example 17, wherein the biodegradable superabsorbent particles comprise polymers based on cellulose or starch, in particular wherein the biodegradable superabsorbent particles comprise carboxymethylcellulose.

A1. An absorbent article (10) comprising:
   a liquid permeable topsheet (26);
   a liquid impermeable backsheet (28);

   an absorbent core (30) disposed at least partially between the topsheet (26) and the backsheet (28); and
   a pair of barrier leg cuffs (32), each barrier leg cuff (32) is formed from a cuff material,
   wherein the cuff material comprises hydrophobic cellulosic fibers.
A2. The absorbent article (10) of example A1, wherein the hydrophobic cellulosic fibers comprise or consist of viscose, lyocell or combinations thereof.
A3. The absorbent article (10) of any one of examples A1-A2, wherein the hydrophobic cellulosic fibers are treated to be hydrophobic.
A4. The absorbent article (10) of example A3, wherein the hydrophobic cellulosic fibers are treated with biodegradable hydrophobic additive .
A5. The absorbent article (10) of any one of examples A1-A4, wherein the cuff material has a basis weight of from 25 gsm to 60 gsm, preferably from 30 gsm to 50 gsm according to the Basis Weight Test method disclosed herein.
A6. The absorbent article (10) of any one of examples A1-A5, wherein the cuff material has a water resistance of at least 6 mbar, preferably at least 8 mbar, more preferably at least 9 mbar, even more preferably at least 10 mbar or even at least 13 mbar according to the Water Resistance Test method disclosed herein.
A7. The absorbent article (10) of any one of examples A1-A6, wherein the cuff material has a Biodegradability of at least 80 %, preferably at least 90 %, more preferably at least 95 %, even more preferably at least 98 % or even at least 99 % according to the Biodegradability Test method disclosed herein.
A8. The absorbent article (10) of any one of examples A1-A7, wherein the cuff material has a machine direction (MD) tensile peak strength of at least 6.0 N/cm, preferably at least 7.0 N/cm, more preferably at least 8.0 N/cm, even more preferably at least 9.0 N/cm or even at least 9.0 N/cm according to the Tensile Strength Test method disclosed herein.
A9. The absorbent article (10) of any one of examples A1-A8, wherein the cuff material has a cross-machine direction (CD) tensile peak strength of at least 2.0 N/cm, preferably at least 2.5 N/cm, more preferably at least 3.0 N/cm, even more preferably at least 3.5 N/cm or even at least 4.0 N/cm according to the Tensile Strength Test method disclosed herein.
A10. The absorbent article (10) of any one of examples A1-A9, wherein the cuff material has a machine direction (MD) peak elongation of at least 15 %, preferably at least 20 %, more preferably at least 25 % or even at least 30 % according to the Tensile Strength Test method disclosed herein.
A11. The absorbent article (10) of any one of examples A1-A10, wherein the cuff material has a cross-machine direction (CD) peak elongation of at least 20 %, preferably at least 40 %, more preferably at least 60 %, even more preferably at least 60 % or even at least 100 % according to the Tensile Strength Test method disclosed herein.
A12. The absorbent article (10) of any one of examples A1-A11, wherein the absorbent article has a Biodegradability of at least 70 %, preferably at least 80 %, more preferably at least 90 %, even more preferably at least 95 %, even more preferably at least 98 % or even at least 99 % according to the Biodegradability Test method disclosed herein.
A13. The absorbent article (10) of any one of examples A1-A12, wherein the topsheet (26) has a Biodegradability of at least 80 %, preferably at least 90 %, more preferably at least 95 %, even more preferably at least 98 % or even at least 99 % according to the Biodegradability Test method disclosed herein.
A14. The absorbent article (10) of any one of examples A1-A13, wherein the backsheet (28) has a Biodegradability of at least 80 %, preferably at least 90 %, more preferably at least 95 %, even more preferably at least 98 % or even at least 99 % according to the Biodegradability Test method disclosed herein.
A15. The absorbent article (10) of any one of examples A1-A14, wherein the absorbent core (30) has a Biodegradability of at least 80 %, preferably at least 90 %, more preferably at least 95 %, even more preferably at least 98 % or even at least 99 % according to the Biodegradability Test method disclosed herein.
A16. The absorbent article (10) of any one of examples A1-A15, wherein the absorbent article comprises a biodegradable acquisition material (38), preferably wherein the biodegradable acquisition materials (38) is a crosslinked cellulosic material, in particular a cellulosic material crosslinked with citric acid.
A17. The absorbent article (10) of any one of examples A1-A15, wherein the absorbent article comprises biodegradable superabsorbent particles.
A18. The absorbent article (10) of example A17, wherein the biodegradable superabsorbent particles comprise polysaccharide polymer, derivatives of polysaccharide polymer, polyaspartate, polyglutamate and/ or the respective salts thereof
A19. The absorbent article (10) of example A17, wherein the biodegradable superabsorbent particles comprise polymers based on cellulose or starch, in particular wherein the biodegradable superabsorbent particles comprise carboxymethylcellulose.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present disclosure have been illustrated and described, it would be obvious to those skilled in the art that various other changes and modifications can be made without departing from the spirit and scope of the present disclosure. It is therefore intended to cover in the appended claims all such changes and modifications that are within the scope of this present disclosure.

## Claims

1. An absorbent article (10) comprising:
a liquid permeable topsheet (26);
a liquid impermeable backsheet (28);
an absorbent core (30) disposed at least partially between the topsheet (26) and the backsheet (28); and
a pair of barrier leg cuffs (32), each barrier leg cuff (32) is formed from a cuff material,
wherein the cuff material comprises hydrophobic cellulosic fibers.

2. The absorbent article (10) of claim 1, wherein the hydrophobic cellulosic fibers comprise or consist of viscose, lyocell or combinations thereof.

3. The absorbent article (10) of any one of the preceding claims, wherein the hydrophobic cellulosic fibers are treated to be hydrophobic.

4. The absorbent article (10) of claim 3, wherein the hydrophobic cellulosic fibers are treated with biodegradable hydrophobic additive.

5. The absorbent article (10) of any one of the preceding claims, wherein the cuff material has a basis weight of from 25 gsm to 60 gsm, preferably from 30 gsm to 50 gsm according to the Basis Weight Test method disclosed herein.

6. The absorbent article (10) of any one of the preceding claims, wherein the cuff material has a water resistance of at least 6 mbar, preferably at least 8 mbar, more preferably at least 9 mbar, even more preferably at least 10 mbar or even at least 13 mbar according to the Water Resistance Test method disclosed herein.

7. The absorbent article (10) of any one of the preceding claims, wherein the cuff material has a Biodegradability of at least 80 %, preferably at least 90 %, more preferably at least 95 %, even more preferably at least 98 % or even at least 99 % according to the Biodegradability Test method disclosed herein.

8. The absorbent article (10) of any one of the preceding claims, wherein the cuff material has a machine direction (MD) tensile peak strength of at least 6.0 N/cm, preferably at least 7.0 N/cm, more preferably at least 8.0 N/cm, even more preferably at least 9.0 N/cm or even at least 9.0 N/cm according to the Tensile Strength Test method disclosed herein.

9. The absorbent article (10) of any one of the preceding claims, wherein the cuff material has a cross-machine direction (CD) tensile peak strength of at least 2.0 N/cm, preferably at least 2.5 N/cm, more preferably at least 3.0 N/cm, even more preferably at least 3.5 N/cm or even at least 4.0 N/cm according to the Tensile Strength Test method disclosed herein.

10. The absorbent article (10) of any one of the preceding claims, wherein the cuff material has a machine direction (MD) peak elongation of at least 15%, preferably at least 20 %, more preferably at least 25 % or even at least 30 % according to the Tensile Strength Test method disclosed herein.

11. The absorbent article (10) of any one of the preceding claims, wherein the cuff material has a cross-machine direction (CD) peak elongation of at least 20 %, preferably at least 40 %, more preferably at least 60 %, even more preferably at least 60 % or even at least 100 % according to the Tensile Strength Test method disclosed herein.

12. An absorbent article (10) comprising:
a liquid permeable topsheet (26);
a liquid impermeable backsheet (28);
an absorbent core (30) disposed at least partially between the topsheet (26) and the backsheet (28); and
a pair of barrier leg cuffs (32), each barrier leg cuff (32) formed from a cuff material,
wherein the cuff material has a water resistance of at least 6 mbar, preferably at least 8 mbar, more preferably at least 9 mbar, even more preferably at least 10 mbar or even at least 13 mbar according to the Water Resistance Test method disclosed herein and
wherein the cuff material has a Biodegradability of at least 80 %, preferably at least 90 %, more preferably at least 95 %, even more preferably at least 98 % or even at least 99 % according to the Biodegradability Test method disclosed herein.

13. The absorbent article (10) of claim 12, wherein the cuff material comprises hydrophobic cellulosic fibers.

14. The absorbent article (10) of claims 12 or 13, wherein the hydrophobic cellulosic fibers comprise or consist of viscose, lyocell or combinations thereof.

15. The absorbent article (10) of any one of claims 12-14, wherein the hydrophobic cellulosic fibers are treated to be hydrophobic, preferably treated with a biodegradable hydrophobic additive.

16. The absorbent article (10) of any one of the preceding claims, wherein the absorbent article has a Biodegradability of at least 70 %, preferably at least 80 %, more preferably at least 90 %, even more preferably at least 95 %, even more preferably at least 98 % or even at least 99 % according to the Biodegradability Test method disclosed herein.
